# EUROPEAN PATENT SPECIFICATION

(11) **EP 1 509 232 B1**
(45) Date of publication and mention of the grant of the patent: **19.11.2008**
(21) Application number: 03724895.2
(22) Date of filing: 22.05.2003
(51) Int. Cl.: A61K 31/55, A61K 31/13, A61P 25/28

(54) **A COMBINATION OF AN NMDA-ANTAGONIST AND ACETYLCHOLINE ESTERASE INHIBITORS FOR THE TREATMENT OF ALZHEIMER'S DISEASE**
KOMBINATION AUS EINEM NMDA-ANTAGONIST UND ACETYLCHOLINESTERASE-HEMMERN ZUR BEHANDLUNG DER ALZHEIMER-KRANKHEIT
COMBINAISON D'UN ANTAGONISTE DE N-METHYL D-ASPARTATE ET D'INHIBITEURS DE L'ESTERASE D'ACETYLCHOLINE POUR LE TRAITEMENT DE LA MALADIE D'ALZHEIMER

(30) Priority: 31.05.2002 DK 200200844; 31.05.2002 US 384467 P
(43) Date of publication of application: 02.03.2005
(73) Proprietor: H. LUNDBECK A/S, 2500 Valby-Copenhagen (DK)
(72) Inventor: THOMSEN, Lars, Lykke, DK-2840 Holte (DK); PEDERSEN, Anders, Gersel, DK-2920 Charlottenlund (DK)
(74) Representative: Kjerrumgaard, Lars Bo
(86) International application number: PCT/DK2003/000342
(87) International publication number: WO 2003/101458

(56) References cited:
- HARTMANN SUSANNE ET AL.: 'Tolerability of memantine in combination with cholinesterase inhibitors in dementia therapy' INTERNATIONAL CLINICAL PSYCHOPHARMACOLOGY vol. 18, no. 2, 2003, pages 81 - 85, XP002967315
- WENK GARY L. ET AL.: 'No interaction of memantine with acetyl - cholinesterase inhibitors approved for clinical use' LIFE SCIENCES vol. 66, no. 12, 2000, pages 1079 - 1083, XP001022849
- REISBERG B. ET AL: 'Memantine in Moderate-to-Severe Alzheimer's Disease' THE NEW ENGLAND JOURNAL OF MEDICINE vol. 348, no. 14, 03 April 2003, pages 1333 - 1341
- PIERRE N. TARIOT ET AL: 'Memantine Treatment in Patients with moderate to severe Alzheimer disease already receiving Donepezil' JAMA vol. 291, no. 3, 21 January 2004, pages 317 - 324
- GAUTHIER S. ET AL: 'Effects of memantine on behavioural symptoms in Alzheimer's disease patients' INTERNATIONAL JOURNAL OF GERIATRIC PSYCHIATRY vol. 20, 2005, pages 459 - 464

## Description

The present invention provides a treatment for mild cognitive impairment (MCI) and for dementia of different types such as dementia of Alzheimer's type,by administration of medicaments having dual mechanisme of action.

Dementia of different origin are a growing problem in the world. Among the elderly, Alzheimer's disease is the most common type of dementia. The prevalence of the disease raises from 2% of the people aged 65-70 to as high as 20% of the people aged 80 and older. Though perhaps not the only contributing factor, the increased life expectancy and the increased elderly population explains the raise in the frequency of the disease.

Alzheimer's disease is a slowly progressing neurodegenerative disease characterised by significant loss of function in more than one cognitive domain. Associated diseases such as psychiatric illness and change in behaviour or personality are common.

Presently, the disease cannot be cured. Current treatment gives for some patients a delay in the symptoms, for others a modest cognitive improvement and a dramatic improvement in only a small number of patients. A slower progression of the disease is also desirable for improving the life quality for the patient and the patient's relatives. However, experience with the current treatment with Alzheimer's therapy, still 30% of the patients do not respond to the treatment. Consequently, a great need for improvement in the treatment of Alzheimer's disease exists.

The mechanisms behind the different types of dementia, including Alzheimer's disease, are not fully understood. Of medicaments available for treatment, which presently is only slowing the progression of the disease, they represent different mechanism of action in the central nervous system.

One group of medicaments are represented by the acetylcholinesterase inhibitors, of which medicaments like Donepezil, Rivastigmine, Galantamine and Tacrine are pharmaceuticals having this particular activity. The acetylcholinesterase inhibitors are presently approved in many countries for treatment of mild to moderate Alzheimer's disease.

Another group of medicaments are the NMDA antagonist of which Memantine is a representative. Memantine was recently approved in the EU for treatment of moderate severe to severe Alzheimer's disease.

Though of very different mechanisms of action, both types of medicaments are useful in the treatment of Alzheimer's disease, though in different stages of the disease progression.

The invention thus provides the combined treatment of a patient suffering from a mild cognitive impairment or dementia on connection with moderate to severe Alzheimer's with a first component which is an acetylcholinesterase inhibitor(s) and a second component which is an NMDA antagonist.

The acetylcholinesterase inhibitors include, but are not limited to:
Donepezil, a known compound described in EP 296560 and US 4895841.
Galantamines use in the treatment of Alzheimer's disease is described in EP236684 and US 4663318.
Rivastigmine as described in national applications corresponding to US 5602176 and GB 2203040.
Tacrine as used in the treatment of cholinergic deficit state, such as Alzheimer's disease, is described in EP 328535 and US 4816456.

Similarly, when the invention is regarded in its broadest sense, the second component is a compound which functions as an NMDA antagonist or partial antagonist of which assays like Ebert et. al European Journal of Pharmacology 1997, 333, 99-104 exists for determining this activity. Other compounds than the compounds mentioned here have the desired effect. It is intended to include compounds which show antagonisme in the assay described above.

In the present invention, the combination of one compound of the group of acetylcholinesterase inhibitors with one compound of the group of NMDA antagonist is included. Likewise, the combination of two compounds of the first group with one or two or the compounds selected from the second group is also within the present invention and *vice versa.*

While all combinations of first and second group compounds are useful the following combinations are considered as the preferred combinations:
Memantine/Donepezil, Memantine/Galantamine, Memantine/Rivastigmine and Memantine/Tacrine.

Where the compounds exists as different polymorphs, isomers, enantiomers or tautomers the present invention also embraces these variations as well as different salts or solvates etc.

Active metabolites of the compounds described are also embraced by the invention.

### Alzheimer's Disease

Characteristics of Alzheimer's Disease are some of the following symptoms occuring:
- Dementia
- Deficits in cognition (such as language, memory, motor skills and perception ie. aphasia, apraxia, agnosia)
- Progressive worsening of memory and cognitive functions

Some of the following associated symptoms often occur:
Depression, insomnia, incontinence, delusions, illusion, hallucinations, emotional or physical outbursts, shouting, wandering, aggression, agitation, apathy, abnormal eating, sexual disorders and weight loss.

Increased motor tone, myoclonus or gait disorder in the late stages of the disease progression.

Since Alzheimer's disease is not curable at present, the complexity of the disease progression and the associated symptoms often gives rise to a multiplicity of diseases, which all need medical treatment. Such treatment regimens and side-effects are difficult to administerable for the patient as well as for the health carer.

Consequently, a positive outcome of an Alzheimer's treatment is an improved cognitive function. A slower progression of the disease, or a delay in the normal disease progression is a positive outcome of a treatment.

Improvements could also be measured in the secondary or associated symptoms of more psychiatric character. Diminished intake or complete stop in the intake of for example antipsychotic, antidepressive, tranquilising or sedative medication will also be signs of a positive response to the treatment of the underlying cause of the dementia, ie. the Alzheimer's Disease.

When combination treatment is evaluated a synergistic effect of the combined administration could be evaluated and the total dose of the combination treatment of individual compounds relative to the doses used for single compound administration could be lowered.

The present invention covers treatment of mild cognitive impairment and dementia in connection with moderate to severe Alzheimer's disease.

In the context of this invention, mild cognitive impairment are characterised by symptoms defined by Petersen et. al.

### Pharmaceutical compositions:

To prepare the pharmaceutical compositions of this invention, an effective amount of the active ingredients, in acid or base addition salt form or base form, is combined in intimate admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included.

It is especially advantageous to formulate the aforementioned pharmaceutical compositions in dosage unit form for ease of administration and uniformity of dosage. Dosage unit form as used in the specification and claims herein refer to physically discrete units suitable as unitary dosages, each unit containing a predetermined quantity of active ingredient calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The NMDA antagonist may be administered before, during or after the administration of the acetylcholinesterase inhibitor provided that the time between the administration of the acetylcholinesterase inhibitor and the administration of the acetylcholinesterase inhibitor is such that ingredients are allowed to act synergistically on the CNS.

When simultaneous administration of NMDA antagonist and an acetylcholinesterase inhibitor is envisaged, a composition containing both an acetylcholinesterase inhibitor and an NMDA antagonist may be particularly convenient. The compositions may be prepared as described herein above.

Simultaneous administration may also be accomplished by administration of the active ingredients in two separate unit dosage forms.

When sequential administration of the NMDA antagonist is envisaged, the pharmaceutical composition may comprise, for example, a kit including discrete unit dosage forms containing the NMDA antagonist and discrete unit dosage forms containing an acetylcholinesterase inhibitor, all contained in the same container or pack, e.g. a blister pack.

### Dose Ranges

The selection of dosage of the first and second component is that which gives the patient relief of the symptoms of the disease. The dosage depends on several factors such as the potency of the selected compounds, the mode of administration, the age and weight of the patient, the severity of the condition to be treated and the like. This is considered to be the skill of the artisan and suitable literature can be consulted for the dosages recommended for each compound.

The dosage ranges for the NMDA antagonist, Memantine are 0.1 mg -500mg of active ingredient pr. dosage. More preferred are 1-50 mg and most preferred are 2-25 mg. Presently the preferred dosage administered is 20mg.

The dosage of the second component, the acetylcholinesterase inhibitor, will depend on the dosage of the NMDA antagonist administered or vice versa. The average daily dosage of the acetylcholinesterase inhibitor are from 0.1 mg -500mg of active ingredient pr. dosage. More preferred are 1-50 mg and most preferred are 2-25 mg. Presently, the dosage regime for the available acetylcholinesterase inhibitor are the following:

| | |
|---|---|
| Tacrine | 10-40 mg four times a day |
| Donepezil | 5-10 mg per day |
| Rivastigmine | 3-12 mg per day |
| Galantamine | 4-24 mg per day |

## Claims

1. The use of a composition comprising:
(a) an effective amount of one or more acetylcholinesterase inhibitor(s) or a pharmaceutically acceptable salt thereof and
(b) an effective amount of one or more NMDA- antagonist(s) or a pharmaceutically acceptable salt thereof
for the manufacture of a medicament for the treatment of mild cognitive impairment or dementia in connection with moderate to severe Alzheimer's.

2. The use according to claim 1 wherein component (a) is selected from the group consisting of Tacrine, Donepezil, Rivastigmine and Galantamine and mixtures thereof.

3. The use according to claims -1-2 wherein component (a) is Donepezil.

4. The use according to claims 1-3 wherein component (b) is Memantine

## Patentansprüche

1. Verwendung einer Zusammensetzung, umfassend:
(a) eine wirksame Menge eines oder mehrerer Acetylcholinesterasehemmer oder eines pharmazeutisch akzeptablen Salzes davon und
(b) eine wirksame Menge eines oder mehrerer NMDA-Antagonisten oder eines pharmazeutisch akzeptablen Salzes davon
für die Herstellung eines Medikaments zur Behandlung einer leichten kognitiven Beeinträchtigung oder Demenz in Verbindung mit mittlerem bis schwerem Alzheimer.

2. Verwendung gemäß Anspruch 1, worin der Bestandteil (a) ausgewählt ist aus der Gruppe, bestehend aus Tacrin, Donepezil, Rivastigmin und Galantamin und Mischungen davon.

3. Verwendung gemäß den Ansprüchen 1 bis 2, worin der Bestandteil (a) Donepezil ist.

4. Verwendung gemäß den Ansprüchen 1 bis 3, worin der Bestandteil (b) Memantin ist.

## Revendications

1. Utilisation d'une composition comprenant :
(a) une quantité efficace d'un ou de plusieurs inhibiteurs de l'acétylcholine estérase ou d'un sel pharmaceutiquement acceptable de celui-ci et
(b) une quantité efficace d'un ou de plusieurs antagonistes de NMDA ou d'un sel pharmaceutiquement acceptable de celui-ci
en vue de la fabrication d'un médicament pour le traitement de la déficience cognitive légère ou de la démence en relation avec un Alzheimer modéré à grave.

2. Utilisation selon la revendication 1, dans laquelle le composant (a) est choisi dans le groupe constitué par la tacrine, le donepezil, la rivastigmine et la galantamine et leurs mélanges.

3. Utilisation selon les revendications 1-2, dans laquelle le composant (a) est le donepezil.

4. Utilisation selon les revendications 1 - 3 dans laquelle le composant (b) est la mémantine.
